(19) 

(11) **EP 4 806 877 A1**

(12) **EUROPEAN PATENT APPLICATION**
published in accordance with Art. 153(4) EPC

(43) Date of publication:
**16.09.2026 Bulletin 2026/38**

(21) Application number: **24888375.3**

(22) Date of filing: **04.09.2024**

(51) International Patent Classification (IPC):
***C07C 17/25*** *(2006.01)* ***C07B 61/00*** *(2006.01)*
***C07C 21/18*** *(2006.01)* ***C07C 21/185*** *(2006.01)*

(52) Cooperative Patent Classification (CPC):
**C07B 61/00; C07C 17/25; C07C 19/08; C07C 21/18; C07C 21/185**

(86) International application number:
**PCT/JP2024/031785**

(87) International publication number:
**WO 2025/100073 (15.05.2025 Gazette 2025/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.11.2023 JP 2023190304**

(71) Applicant: **AGC Inc.**
**Chiyoda-ku**
**Tokyo 100-8405 (JP)**

(72) Inventors:
- **FURUTA, Shoji**
  **Tokyo 100-8405 (JP)**
- **MATSUNAMI, Asuka**
  **Tokyo 100-8405 (JP)**

(74) Representative: **Müller-Boré & Partner Patentanwälte PartG mbB**
**Friedenheimer Brücke 21**
**80639 München (DE)**

(54) **METHOD FOR PRODUCING HALOGENATED ALKENE**

(57) A method of producing a halogenated alkene includes converting a halogenated alkane containing a fluorine atom and having from 2 to 4 carbon atoms into a halogenated alkene containing a fluorine atom and having from 2 to 4 carbon atoms in a gas phase in the presence of silicon oxide and an alkali metal element.

EP 4 806 877 A1

## Description

Technical Field

The present disclosure relates to a method of producing a halogenated alkene.

Background Art

In recent years, halogenated alkenes (i.e., fluoroolefins) have been attracting attention as compounds having a small global warming potential.

For example, Patent Document 1 describes a method of producing a hydrofluoroolefin in which a hydrofluorocarbon is converted into a hydrofluoroolefin in the presence of a fluorine-containing compound having a normal boiling point higher than the normal boiling point of the target hydrofluoroolefin. The reaction process of this production method includes a process of bringing a hydrofluorocarbon into contact with a catalyst. Specifically, alumina ($Al_2O_3$) is used as the catalyst.

[Prior Art Documents]

[Patent Documents]

Patent Document 1: International Publication No. 2017/104829

SUMMARY OF INVENTION

Technical Problem

However, in a case in which a fluoroolefin is produced by a dehydrofluorination reaction of a fluorocarbon using a catalyst such as that described in Patent Document 1, the catalyst deteriorates over time due to the generated hydrogen fluoride, and the production amount of the fluoroolefin decreases over time.

Therefore, an object in one embodiment in the present disclosure is to provide a method of producing a halogenated alkene in which a decrease in production amount over time is suppressed as compared with conventional methods.

Solution to Problem

The present disclosure includes the following aspects.

<1> A method of producing a halogenated alkene, the method including converting a halogenated alkane containing a fluorine atom and having from 2 to 4 carbon atoms into a halogenated alkene containing a fluorine atom and having from 2 to 4 carbon atoms in a gas phase in the presence of silicon oxide and an alkali metal element.

<2> The method of producing a halogenated alkene according to <1>, in which the halogenated alkane includes a halogenated alkane represented by the following Formula (1), and the halogenated alkene includes a halogenated alkene represented by the following Formula (2):

$$CR^1R^2X^1\text{-}CR^3R^4X^2 \qquad (1)$$

$$CR^1R^2{=}CR^3R^4 \qquad (2)$$

in which, in Formula (1) and Formula (2), $R^1$ to $R^4$ are each independently a hydrogen atom, a fluorine atom, a methyl group, a fluorinated methyl group, an ethyl group, or a fluorinated ethyl group, a total number of fluorine atoms in $R^1$ to $R^4$ is 1 or more, and the number of carbon atoms is from 2 to 4, and in which in Formula (1), one of $X^1$ or $X^2$ is a hydrogen atom, and another is a fluorine atom.

<3> The method of producing a halogenated alkene according to <1> or <2>, in which silicon tetrafluoride is produced.

<4> The method of producing a halogenated alkene according to any one of <1> to <3> the method including:

producing the halogenated alkene and hydrogen fluoride by a dehydrofluorination reaction of the halogenated alkane in a gas phase; and

producing silicon tetrafluoride by a reaction between the produced hydrogen fluoride and silicon oxide.

<5> The method of producing a halogenated alkene according to any one of <1> to <4>, in which the halogenated alkane is at least one selected from the group consisting of 1,1-difluoroethane, 1,2-difluoroethane, 1,1,1-trifluoroethane, 1,1,2-trifluoroethane, 1,1,2,2-tetrafluoroethane, 1,1,1,2-tetrafluoroethane, and 1,1,1,2,2-pentafluoroethane.

<6> The method of producing a halogenated alkene according to any one of <1> to <5>, in which the halogenated alkene is at least one selected from the group consisting of fluoroethylene, 1,1-difluoroethylene, 1,2-difluoroethylene, trifluoroethylene, and tetrafluoroethylene.

<7> The method of producing a halogenated alkene according to any one of <1> to <6>, in which the halogenated alkane is converted in the presence of a diluent gas.

<8> The method of producing a halogenated alkene according to <7>, in which the diluent gas is at least one selected from the group consisting of nitrogen, hydrogen, carbon dioxide, helium, ethane, propane, isobutane, n-butane, propylene, and fluorinated methane.

<9> The method of producing a halogenated alkene according to any one of <1> to <8>, in which the halogenated alkane is converted at a temperature of from 400 to 1000°C.

<10> A method of producing a halogenated alkene, the method including converting a halogenated alkane containing a fluorine atom and having from 2 to 4 carbon atoms into a halogenated alkene containing a fluorine atom and having from 2 to 4 carbon atoms in a gas phase in the presence of boron oxide.

Advantageous Effects of Invention

According to the present disclosure, it is possible to provide a method of producing a halogenated alkene in which a decrease in production amount over time is suppressed as compared with conventional methods.

DESCRIPTION OF EMBODIMENTS

In the present disclosure, a numerical range indicated using "to" means a range including the numerical values described before and after "to" as the minimum value and the maximum value, respectively.

In the numerical ranges described step-by-step in the present disclosure, the upper limit value or the lower limit value described in a certain numerical range may be replaced with the upper limit value or the lower limit value of another step-by-step numerical range. In addition, in the numerical ranges described in the present disclosure, the upper limit value or the lower limit value described in a certain numerical range may be replaced with the values shown in the Examples.

In the present disclosure, a combination of two or more preferred embodiments is a more preferred embodiment.

In the present disclosure, the amount of each component means the total amount of the plurality of types of substances in a case in which a plurality of types of substances corresponding to each component exist, unless otherwise specified.

[Method of producing a halogenated alkene]

The method of producing a halogenated alkene in the present disclosure includes converting a halogenated alkane containing a fluorine atom and having from 2 to 4 carbon atoms into a halogenated alkene containing a fluorine atom and having from 2 to 4 carbon atoms in a gas phase in the presence of silicon oxide and an alkali metal element.

Hereinafter, the "halogenated alkane containing a fluorine atom and having from 2 to 4 carbon atoms" is also referred to as a "specific halogenated alkane," and the "halogenated alkene containing a fluorine atom and having from 2 to 4 carbon atoms" is also referred to as a "specific halogenated alkene".

According to the method of producing a halogenated alkene in the present disclosure, a decrease in production amount over time is suppressed as compared with conventional methods. The reason for this is not certain, but is presumed as follows.

In a reaction for obtaining a halogenated alkene containing a fluorine atom from a halogenated alkane containing a fluorine atom, hydrogen fluoride is generated. For example, the generated hydrogen fluoride reacts with alumina to generate $AlF_3$ in a case in which alumina ($Al_2O_3$) is used as a catalyst, and generates $CaF_2$ in a case in which calcium carbonate ($CaCO_3$) is used as a catalyst. Here, since the boiling point of $AlF_3$ is 1260°C and the boiling point of $CaF_2$ is 2533°C, these are solids in the reaction system. Therefore, the generated $AlF_3$ and $CaF_2$ remain in the reaction system and coat the reaction sites present on the surface of the catalyst. By coating the reaction sites of the catalyst, the activity of the catalyst rapidly decreases.

In contrast, in the method of producing a halogenated alkene in the present disclosure, a specific halogenated alkene is obtained from a specific halogenated alkane in the presence of silicon oxide and an alkali metal element. At this time, silicon tetrafluoride ($SiF_4$) is generated, regardless of the reaction scheme, whether the generated hydrogen fluoride reacts with silicon oxide or the specific halogenated alkane directly reacts with silicon oxide and the alkali metal element.

Since the boiling point of silicon tetrafluoride is -95°C, it is a gas in the reaction system and is released out of the reaction system. Therefore, in the method of producing a halogenated alkene in the present disclosure, it is considered that coating of the silicon oxide is suppressed, and a rapid decrease in the production amount of the halogenated alkene is suppressed.

Hereinafter, the method of producing a halogenated alkene in the present disclosure will be described in detail.

(Halogenated alkane)

In the method of producing a halogenated alkene in the present disclosure, a specific halogenated alkane is used as a raw material.

The number of carbon atoms of the specific halogenated alkane is from 2 to 4, and the number of carbon atoms may be 2, 3, or 4. From the viewpoint of the boiling point range of a compound that can be a refrigerant, the number of carbon atoms of the specific halogenated alkane is preferably 2 or 3.

The specific halogenated alkane contains a fluorine atom. The number of fluorine atoms in the specific halogenated alkane is preferably 2 or more.

The number of hydrogen atoms in the specific halogenated alkane is preferably 1 or more.

The specific halogenated alkane may contain one or more other halogen atoms other than a fluorine atom. Examples of the one or more other halogen atoms include a chlorine atom, a bromine atom, and an iodine atom, and a chlorine atom is preferred. The specific halogenated alkane may not contain any other halogen atoms.

Examples of the specific halogenated alkane include a halogenated alkane represented by the following Formula (1).

$$CR^1R^2X^1\text{-}CR^3R^4X^2 \qquad (1)$$

In Formula (1), $R^1$ to $R^4$ are each independently a hydrogen atom, a fluorine atom, a methyl group, a fluorinated methyl group, an ethyl group, or a fluorinated ethyl group, a total number of fluorine atoms in $R^1$ to $R^4$ is 1 or more, the number of carbon atoms is from 2 to 4, and one of $X^1$ or $X^2$ is a hydrogen atom, and another is a fluorine atom.

It is preferable that $R^1$ and $R^3$ are each independently a hydrogen atom or a fluorine atom, and it is preferable that $R^2$ and $R^4$ are a hydrogen atom, a fluorine atom, $CH_3$, $CH_2F$, $CHF_2$, or $CF_3$.

Examples of the halogenated alkane represented by Formula (1) include the following compounds.

$CHF_2CH_3$: 1,1-difluoroethane (HFC-152a)
$CH_2FCH_2F$: 1,2-difluoroethane (HFC-152)
$CF_3CH_3$: 1,1,1-trifluoroethane (HFC-143a)
$CHF_2CH_2F$: 1,1,2-trifluoroethane (HFC-143)
$CF_3CH_2F$: 1,1,1,2-tetrafluoroethane (HFC-134a)
$CHF_2CHF_2$: 1,1,2,2-tetrafluoroethane (HFC-134)
$CF_3CHF_2$: 1,1,1,2,2-pentafluoroethane (HFC-125)

The specific halogenated alkane may contain other halogenated alkanes (provided that they contain a fluorine atom and have from 2 to 4 carbon atoms) other than the halogenated alkane represented by Formula (1). A proportion of the halogenated alkane represented by Formula (1) to a total amount of the specific halogenated alkane is preferably 30% by mol or more, and more preferably 50% by mol or more.

(Halogenated alkene represented by Formula (2))

In the method of producing a halogenated alkene in the present disclosure, a specific halogenated alkene is obtained as a reaction product.

The number of carbon atoms of the specific halogenated alkene is from 2 to 4, and the number of carbon atoms may be 2, 3, or 4.

The specific halogenated alkene contains a fluorine atom. The number of fluorine atoms in the specific halogenated alkene is 1 or more.

The specific halogenated alkene may contain one or more other halogen atoms other than a fluorine atom. Examples of the one or more other halogen atoms include a chlorine atom, a bromine atom, and an iodine atom, and a chlorine atom is preferred. The specific halogenated alkene may not contain any other halogen atoms.

Examples of the specific halogenated alkene include a halogenated alkene represented by the following Formula (2).

$$CR^1R^2=CR^3R^4 \qquad (2)$$

In Formula (2), $R^1$ to $R^4$ are each independently a hydrogen atom, a fluorine atom, a methyl group, a fluorinated methyl group, an ethyl group, or a fluorinated ethyl group, a total number of fluorine atoms in $R^1$ to $R^4$ is 1 or more, and the number of carbon atoms is from 2 to 4.

It is preferable that $R^1$ and $R^3$ are each independently a hydrogen atom or a fluorine atom, and it is preferable that $R^2$ and $R^4$ are a hydrogen atom, a fluorine atom, $CH_3$, $CH_2F$, $CHF_2$, or $CF_3$.

Examples of the halogenated alkene represented by Formula (2) include the following compounds.

$CHF=CH_2$: fluoroethylene (HFO-1141)
$CF_2=CH_2$: 1,1-difluoroethylene (HFO-1132a)
CHF=CHF: 1,2-difluoroethylene (HFO-1132(E), HFO-1132(Z))
$CHF=CF_2$: trifluoroethylene (HFO-1123)
$CF_2=CF_2$: tetrafluoroethylene (FO-1114)

Among them, the halogenated alkene represented by Formula (2) is preferably at least one selected from the group consisting of HFO-1132, HFO-1132a, and HFO-1123 from the viewpoint of usefulness as a refrigerant composition. In addition, HFO-1141 and FO-1114 are preferred from the viewpoint of usefulness as a resin.

(Silicon oxide and alkali metal element)

In the method of producing a halogenated alkene in the present disclosure, a specific halogenated alkane is converted into a specific halogenated alkene in the presence of silicon oxide and an alkali metal element.

The silicon oxide and the alkali metal element may be an integrated compound or composite containing both, or separate substances separately containing the silicon oxide and the alkali metal element may be used, and two or more of these may be used in combination. Examples thereof include glass, sodium silicate, sodium silicate cullet and the like, which contain silicon oxide and an oxide of an alkali metal; a composite in which an alkali metal-containing compound is supported on a silicon oxide particle; and a combination of a silicon oxide particle and an alkali metal-containing compound.

In a case in which the silicon oxide and the alkali metal element are integrated, uneven distribution in the reaction system is easily suppressed. In a case in which silicon oxide and an alkali metal-containing compound are used as separate substances, it is easy to prepare those with high purity, and the generation of unnecessary by-products when used in the reaction is easily suppressed.

Hereinafter, compounds and composites containing both silicon oxide and an alkali metal element, and those containing silicon oxide and an alkali metal element as separate substances are also collectively referred to as a "reactant".

The reactant may contain other components other than silicon oxide and the alkali metal element. Examples of other components include calcium, aluminum, magnesium, iron, boron, lead, zinc, and the like.

A shape of the glass is not particularly limited, and may be any of an irregular shape such as a pulverized product, a cullet shape, a flake shape, a spherical shape, or the like. Further, it may be formed into a pellet shape, a hollow shape, a cylindrical shape, or the like. These shapes may be appropriately combined.

Examples of the silicon oxide particle in the composite or the combination as separate substances include silica sand, quartz, diatomaceous earth, colloidal silica, precipitated silica, silica gel, fumed silica, rice husk and the like, and silica sand is preferred from the viewpoint of purity and price.

A shape of the silicon oxide particle is not particularly limited, and may be any of an irregular shape such as a natural product or a pulverized product, a cullet shape, a flake shape, a spherical shape, or the like. Further, it may be formed into a pellet shape, a hollow shape, a cylindrical shape, or the like. Further, the silicon oxide particle may have a pore structure (porous or the like). These shapes may be appropriately combined, and examples thereof include a porous cylindrical molded product.

The silicon oxide particle preferably has a low impurity content, and an impurity content of the silicon oxide particle is preferably low from the viewpoint of suppressing the generation of unnecessary by-products, and a content of silicon oxide in the silicon oxide particle is preferably 70% by mass or more, more preferably 80% by mass or more, and even more preferably 90% by mass or more.

Regarding a size of the glass, the composite and the silicon oxide particle, an average particle diameter is preferably 20 μm or more, and more preferably 50 μm or more, from the viewpoint of suppressing clogging in the reactor. Regarding the size of the glass, the composite, and the silicon oxide particle, the average particle diameter is preferably 10 mm or less, more preferably 5 mm or less, and even more preferably 1 mm or less, from the viewpoint of securing a surface area serving as a reaction site.

The average particle diameter of the glass, the composite, and the silicon oxide particle is determined as a

particle diameter (D50) at which a cumulative total is 50% in a volume-based weight cumulative particle size distribution curve obtained by measurement with a Coulter counter. The aperture diameter is appropriately set according to the particle diameter range of the measurement target.

A silicon content in the reactant may be 1 atomic% or more, 10 atomic% or more, or 20 atomic% or more. Further, the silicon content in the reactant may be 90 atomic% or less, or 80 atomic% or less. An oxygen content in the reactant may be 1 atomic% or more, 5 atomic% or more, or 10 atomic% or more. The oxygen content in the reactant may be 90 atomic% or less, or 80 atomic% or less.

An alkali metal element content in the reactant may be 1 atomic% or more, 5 atomic% or more, or 8 atomic% or more. The content of the alkali metal element in the reactant may be 90 atomic% or less, or 50 atomic% or less.

The content of each element in the reactant is determined by scanning electron microscopy energy dispersive X-ray spectroscopy (SEM-EDX analysis).

In the reactant, the silicon content (atomic%) is preferably higher than the alkali metal element content (atomic%), and is preferably higher than a total content of alkaline earth metals and Group 13 elements of the periodic table, and it is preferable that the element having the highest content (atomic%) among the elements excluding oxygen is silicon.

Further, in the reactant, the alkali metal element content (atomic%) is preferably higher than the respective contents of elements other than silicon, oxygen and the alkali metal element. In the glass, a content (atomic%) of the alkali metal element may be more than, less than, or the same as the total content of alkaline earth metals and Group 13 elements of the periodic table.

The alkali metal element is preferably at least one selected from the group consisting of Na, K, Rb, and Cs, and from the viewpoint of activity, selectivity, or procurement, is preferably at least one selected from the group consisting of Na, K, and Cs.

The alkali metal-containing compound may simply contain an alkali metal element, and examples thereof include halides such as alkali metal fluorides and chlorides, hydroxides, carbonates, and the like, and specific examples thereof include NaF, KF, CsF, NaOH, KOH, $Na_2CO_3$, $K_2CO_3$, and NaCl.

(Reaction scheme)

In the method of producing a halogenated alkene in the present disclosure, silicon tetrafluoride ($SiF_4$) is produced. In the method of producing a halogenated alkene in the present disclosure, a reaction scheme is conceivable in which, in a gas phase, a specific halogenated alkene and hydrogen fluoride are produced by a dehydrofluorination reaction of a specific halogenated alkane (first process), and silicon tetrafluoride is produced by a reaction between the produced hydrogen fluoride and silicon oxide (second process). The first process and the second process may proceed continuously without being distinguished.

Note that the method of producing a halogenated alkene in the present disclosure may proceed via a reaction scheme other than the above reaction scheme, and for example, silicon oxide or an alkali metal compound may directly react with a halogenated alkane to produce silicon tetrafluoride.

Further, in addition to silicon tetrafluoride, other compounds may be produced.

Below, an assumed example of a reaction scheme is shown in which a halogenated alkene represented by Formula (2) is obtained as a specific halogenated alkene using a halogenated alkane represented by Formula (1) as a specific halogenated alkane.

$$CR^1R^2X^1\text{-}CR^3R^4X^2 \rightarrow CR^1R^2{=}CR^3R^4 + HF$$

$$SiO_2 + 4HF \rightarrow SiF_4 + 2H_2O$$

Since the generated silicon tetrafluoride is a gas, it is released from the reaction system to the outside. Therefore, the influence of by-products on the silicon oxide is suppressed, and a rapid decrease in the production amount of the halogenated alkene is suppressed.

Note that in a conventional production method using alumina, calcium carbonate, or the like as a catalyst, the generated hydrogen fluoride reacts with the catalyst as follows.

$$Al_2O_5 + 6HF \rightarrow 2AlF_3 + 3H_2O$$

$$CaCO_3 + 2HF \rightarrow 2CaF_2 + H_2O + CO_2$$

Since the produced aluminum fluoride ($AlF_3$) and calcium fluoride ($CaF_2$) are solids, they are not released out of the reaction system but remain in the reaction system and coat a surface of alumina and calcium carbonate, which are catalysts. As a result, the catalyst is deactivated because the active sites on the surface are covered; therefore, in the

conventional production method, work for removing the deteriorated catalyst and replacing it with a new one occurs. Therefore, in the conventional production method using alumina, calcium carbonate, or the like as a catalyst, productivity is not stable, and it is necessary to stop the reaction every time the catalyst is replaced.

In contrast, in the method of producing a halogenated alkene in the present disclosure, the work of removing the deteriorated catalyst is reduced, and there is an advantage that productivity can be maintained.

And, in the method of producing a halogenated alkene in the present disclosure, the reaction can be continued by replenishing the consumed silicon oxide. An amount of consumed silicon oxide can be converted from an amount of silicon tetrafluoride released out of the reaction system. Specifically, the amount of released silicon tetrafluoride can be measured by passing the released silicon tetrafluoride through water, an alkaline aqueous solution or the like to form hydrogen fluoride, hexafluorosilicic acid, salts thereof, or the like, and titrating these.

On the other hand, in a conventional production method using alumina or calcium carbonate as a catalyst, it is difficult to estimate the amount of the deteriorated catalyst because $AlF_3$ or $CaF_2$ remains in the reaction system. Therefore, in the conventional production method, it is difficult to appropriately estimate the replenishment amount of the catalyst.

Furthermore, in a case of reacting in a fluidized bed, it is desired that the fluidity of the catalyst does not change significantly; however, in the conventional method, $AlF_3$ or $CaF_2$ adheres to the catalyst, so a weight and density of the catalyst change and the fluidity fluctuates. Therefore, it is difficult to maintain an appropriate fluid state.

In contrast, in the production method in the present disclosure, since the by-product $SiF_4$ is a gas and is released out of the reaction system, there is no significant change in the fluidity of the silicon oxide, and it is easy to maintain an appropriate fluid state.

(Reaction conditions)

The method of producing a halogenated alkene in the present disclosure is performed in a gas phase because the specific halogenated alkane is a gas at room temperature.

In the method of producing a halogenated alkene in the present disclosure, the raw material gas may simply contain a specific halogenated alkane, and may contain components other than the specific halogenated alkane. The raw material gas may consist only of the specific halogenated alkane, or may contain isomers, disproportionation products, impurities, and the like obtained when producing the specific halogenated alkane. From the viewpoint of suppressing side reactions, a content of the specific halogenated alkane is preferably 10% by mol or more, more preferably 30% by mol or more, and even more preferably 50% by mol or more, with respect to a total amount of the raw material gas. A content of the halogenated alkane represented by Formula (1) may be 100% by mol with respect to the total amount of the raw material gas.

As a reactor for reacting the halogenated alkane and the reactant, any reactor that can withstand the temperature and pressure described later can be used, and the shape and structure are not particularly limited. Examples of the reactor include a cylindrical vertical reactor. Examples of a material of the reactor include glass, stainless steel, iron, nickel, chromium, and alloys containing iron, nickel or chromium as a main component. An inside of the reactor may be coated with platinum, gold, or the like. Further, the reactor may be provided with a heater such as an electric heater for heating the inside of the reactor.

The reactant may be accommodated in any form of a fixed bed type, a fluidized bed type, or a moving bed type. In the case of a fixed bed type, it may be either a horizontal fixed bed type or a vertical fixed bed type. The reactor may rotate as a whole.

In addition, the reaction system may be a flow system or a batch system.

In a fixed bed reactor, various molded bodies of a reactant-supported carrier are filled in order to reduce the pressure loss of the reaction fluid. Further, a system in which a reactant is filled as in a fixed bed reactor, moved by its gravity, and extracted from the bottom of the reactor for regeneration or the like is called a moving bed.

In a fluidized bed reactor, an operation is performed so that the reactant layer exhibits fluid-like characteristics by the reaction fluid, so the reactant moves within the reactor while mixing with the reaction fluid.

A fixed bed reactor is preferred in that the choice of a shape of the reactant is wide and abrasion of the reactant can be suppressed, and a fluidized bed reactor is preferred from the viewpoint that the internal temperature becomes uniform and local heating is easily avoided.

The fixed bed reactor includes a tubular reactor and a tank reactor, and a tubular reactor is preferred because of the ease of controlling a reaction temperature. Furthermore, a multi-tube heat exchange reaction or the like, in which a large number of reaction tubes with small tube diameters are arranged in parallel and a heating medium is circulated on the outside, can be adopted. In a case in which a plurality of reactors are provided in series, a plurality of reactant layers will be provided. There may be at least one stage of the reactant layer, or there may be two or more stages.

In the case of a fluidized bed reactor, the raw material gas and further a diluent gas may be circulated from below in the vertical direction, and a product gas may be extracted from above in the vertical direction. The fluidized bed reactor

may be provided with a stirring blade from the viewpoint of further increasing fluidity. Further, from the viewpoint of preventing the flow of gas in the fluidized bed reactor from being biased, a gas distribution plate may be provided in the fluidized bed reactor. A material of the gas distribution plate is not particularly limited, and is preferably composed of a material having low reactivity with the raw material gas, the product gas, and the like. Examples of the material of the gas distribution plate include sintered metal. The size, arrangement position, and number of the gas distribution plates may be appropriately adjusted according to the flow of gas.

In the method of producing a halogenated alkene in the present disclosure, the halogenated alkane is preferably converted at a temperature of from 400 to 1000°C, more preferably converted at a temperature of from 450 to 900°C, and even more preferably converted at a temperature of from 500 to 800°C.

In a case in which conversion is performed at 400°C or higher, the reaction proceeds appropriately, and a conversion rate of the halogenated alkene is improved. On the other hand, in a case in which conversion is performed at 1000°C or lower, a decrease in selectivity due to carbon-carbon bond cleavage of the raw material and a disproportionation reaction of the reaction product (unsaturated compound) are suppressed.

It is also possible to suppress a decrease in the conversion rate by adjusting it to the above temperature range to appropriately maintaining the reaction temperature. Examples of a method of maintaining the reaction temperature in the reactant layer at a desired temperature include a method of heating the reactant layer from the outside with a heating medium, an electric furnace.

As described above, in the method of producing a halogenated alkene in the present disclosure, the reaction can be continued by replenishing a consumed silicon oxide, and productivity can be maintained. From the viewpoint of continuing the reaction, it is preferable to continuously supply a consumed amount of silicon oxide. A supply position of silicon oxide in the reactor is not particularly limited, and may be from the upper part or the lower part of the reactor.

In the method of producing a halogenated alkene in the present disclosure, the raw material gas containing the halogenated alkane may be supplied to the reactor at room temperature, or may be appropriately heated (preheated) before being supplied to the reactor. In a case in which preheating is performed, the raw material gas is preferably heated to 80°C or more and the reaction temperature in the reactor or less before being supplied to the reactor. In a case in which the preheating temperature is 80°C or more, an internal temperature of the reactor is unlikely to decrease, and it is easy to achieve a set conversion rate. Further, in a case in which the preheating temperature is the reaction temperature in the reactor or less, undesirable reactions are suppressed and selectivity is improved.

Since the dehydrofluorination reaction in the present disclosure is a reaction in which the number of molecules increase, the forward reaction becomes disadvantageous in a case in which the pressure is increased.

The pressure when reacting the halogenated alkane and the reactant is not particularly limited, but from the viewpoint of improving the conversion rate, it is preferably from -0.05 to 2 MPa, more preferably from -0.01 to 1 MPa, and even more preferably from normal pressure to 0.5 MPa.

In the present disclosure, pressure means gauge pressure.

A residence time of the halogenated alkane is preferably from 0.5 to 300.0 seconds, more preferably from 1.0 to 100.0 seconds, and even more preferably from 1.5 to 60.0 seconds.

A residence time (seconds) is calculated using the following Formula.

Residence time (seconds) = [Length of filling the reactant in the reactor (cm)] / [Linear velocity (cm/sec)]

The linear velocity means a speed at which the halogenated alkane passes through the reactant per unit time.

Further, an average bulk density of the reactant is preferably 0.05 g/cm$^3$ or more, more preferably 0.1 g/cm$^3$ or more, and even more preferably 0.2 g/cm$^3$ or more. In a case in which the average bulk density of the reactant is 0.05 g/cm$^3$ or more, the conversion rate is improved.

The average bulk density of the reactant is an average value of the density of the reactant when gas is not circulated in the reactor.

The average bulk density of the reactant is measured by a container method. In the container method, the reactant is placed in a container of a known volume until it overflows, and then excess reactant protruding from the rim of the top surface of the container is removed with a spatula or the like, and the mass of the reactant in the container is measured. The bulk density (g/mL) is calculated from the mass of the reactant and the capacity (volume) of the container. This measurement is performed three times, and the average value thereof is taken as the average bulk density.

Conversion of the specific halogenated alkane is preferably performed in the presence of a diluent gas. The diluent gas is preferably at least one selected from the group consisting of nitrogen, hydrogen, carbon dioxide, helium, propane, isobutane, n-butane, ethane, propylene, and fluorinated methane. Examples of the fluorinated methane include monofluoromethane, difluoromethane, trifluoromethane, and monofluoromethane.

A molar ratio of the specific halogenated alkane to the diluent gas in the gas phase is preferably from 0.1 to 5.0, more preferably from 0.5 to 3.0, and even more preferably from 0.5 to 2.0.

In general, in a method of producing a halogenated alkene, a diluent gas is used from the viewpoint of suppressing a disproportionation reaction caused by an increase in concentration of the produced halogenated alkene, and from the viewpoint of concern about explosion due to an increase in concentration depending on the type of halogenated alkene.

In the method of producing a halogenated alkene in the present disclosure, since reactivity can be controlled by residence time, reaction temperature, or the like, the concentration of the specific halogenated alkene in an outlet gas can be controlled by these controls. In the method of producing a halogenated alkene in the present disclosure, since it is possible to maintain the production amount of the halogenated alkene and keep it within a certain range by the above control and the like, the specific halogenated alkane as a raw material can be included in the outlet gas to some extent or more. The specific halogenated alkane in the outlet gas also functions as a diluent. Therefore, in the method of producing a halogenated alkene in the present disclosure, it is also possible to suppress the amount of diluent gas used. Note that the method of producing a halogenated alkene in the present disclosure also includes an embodiment in which no diluent gas is used.

Further, in the method of producing a fluoroolefin using an alumina catalyst, as described in Patent Document 1, if the amount of the diluent is reduced, the conversion rate decreases; therefore, it is difficult to use the raw material gas as a diluent, and use of a diluent gas such as nitrogen or carbon dioxide is essential. Since diluent gases such as nitrogen gas and carbon dioxide have lower boiling points than the halogenated alkene which is a reaction product, or have boiling point ranges close to it, energy is required to separate and purify the diluent gas from the reaction product.

In the method of producing a halogenated alkene in the present disclosure, even if the raw material gas is used as a part or all of the diluent, a decrease in the production amount of the halogenated alkene over time is suppressed. Since the halogenated alkane of the raw material has a high boiling point and its boiling point range is far from that of the halogenated alkene which is a reaction product, an energy load for separation and purification can also be reduced in the method of producing a halogenated alkene in the present disclosure.

From the viewpoint of controlling the efficiency and selectivity of the reaction, the conversion of the specific halogenated alkane is preferably performed in a gas phase in the presence of water, and a concentration of water is preferably less than 500 ppm by volume with respect to a total amount of the raw material gas containing the specific halogenated alkane.

The dehydrofluorination reaction in the present disclosure also generates water. Therefore, it can be said that the reaction proceeds without problems even if water exists in the present system. Further, when hydrogen fluoride is eliminated from the raw material, or when hydrogen fluoride reacts with silicon oxide, the existence of water molecules may allow the reaction to proceed more efficiently via a hydrogen bond network. Therefore, in the dehydrofluorination reaction in the present disclosure, it is possible to add a small amount of water, and it is presumed that there is a possibility of bringing about a favorable effect.

On the other hand, from the viewpoint of suppressing clogging of gas flow paths due to the reaction of the generated silicon tetrafluoride and water near the outlet to generate hexafluorosilicic acid or the like and the precipitation thereof, the concentration of water is preferably less than the above range.

As a general method for measuring the concentration of water in a gas, a method using a commercially available dew point meter can be mentioned. By having the concentration of water be less than 500 ppm by volume with respect to a total amount of the specific halogenated alkane, the conversion rate increases and a target product can be obtained with high selectivity. The concentration of water is preferably 300 ppm by volume or less, more preferably 100 ppm by volume or less, even more preferably 50 ppm by volume or less, and particularly preferably 10 ppm by volume or less, from the point that the conversion rate can be further improved and the target compound can be obtained with even higher selectivity. A lower concentration of water is preferred, but from the viewpoint of a cost of dehydration treatment of the specific halogenated alkane and the diluent gas and the difficulty of process management, it is preferably 0.5 ppm by volume or more, and more preferably 1 ppm by volume or more.

The concentration of water is a content of water contained in the raw material gas when reacting the specific halogenated alkane and the reactant. Note that the concentration of water may be replaced with a content of water contained in the raw material gas before flowing into the reactor.

The method of producing a halogenated alkene in the present disclosure may further include a process of drying the reactant before reacting the specific halogenated alkane and the reactant. By drying the reactant, water contained in the reactant may be removed, and the concentration of water may be adjusted within the above range.

A method of drying the reactant is not particularly limited, and it may be dried before filling the reactor with the reactant, or may be dried after filling the reactor with the reactant. In a case of drying after filling the reactor with the reactant, preheating of the reactor can also be performed together with the drying of the reactant. Specifically, the reactant may be dried by filling the reactor with the reactant and heating the reactor while circulating a diluent gas.

In the present disclosure, a conversion rate is a ratio (%) of a molar amount of the specific halogenated alkane consumed in the reaction to a molar amount of the specific halogenated alkane supplied to the reactor. The molar amount consumed in the reaction of the specific halogenated alkane, which is the raw material, is the difference between a molar

amount of the specific halogenated alkane supplied to the reactor and a molar amount of the specific halogenated alkane contained in the outflow gas from the reactor outlet.

In general, a higher conversion rate is preferred from the viewpoint of productivity. However, in the case of a specific halogenated alkene that has a risk of explosion due to high concentration, it is preferable to select operating conditions such that the conversion rate is 70% or less, from the viewpoint of suppressing explosion and suppressing a disproportionation reaction of the specific halogenated alkene. The conversion rate is preferably 50% or less, and more preferably 30% or less. In a case in which the conversion rate is too low, productivity decreases and the equipment becomes large; therefore, it is preferable to select operating conditions such that the conversion rate is 5% or more. The conversion rate is preferably 10% or more, and more preferably 15% or more.

In the present disclosure, a selectivity means a ratio (% by mol) of a molar amount of the target product contained in the reactor outlet gas to a total molar amount of compounds other than the raw material contained in the reactor outlet gas (provided that it is a compound derived from carbon of the specific halogenated alkane, which is the raw material; compounds such as silicon tetrafluoride not having carbon derived from the raw material are excluded).

The selectivity is preferably 100% since a purification process after the reaction becomes unnecessary, but side reactions can also occur in a reaction temperature region necessary for obtaining a desired conversion rate. A higher selectivity is preferred because an amount of waste can be reduced, an energy load of the purification process after the reaction can be reduced, and a reactant life can be lengthened. The selectivity is preferably 90% or more, more preferably 93% or more, and even more preferably 95% or more.

Examples of a compound other than the raw material compound and the target product contained in the reactor outlet gas include carbon monoxide, carbon dioxide, water, silicon tetrafluoride, and the like.

According to the method of producing a halogenated alkene in the present disclosure, a decrease in the production amount of the specific halogenated alkene is suppressed in production over a long period of time (specifically, 5 hours or more). A production amount of the specific halogenated alkene after the lapse of 5 hours with respect to a production amount of the specific halogenated alkene after the lapse of 1 hour is preferably 50% or more, more preferably 60% or more, and even more preferably 70% or more.

The production amount is confirmed from an area ratio (GC Area%) corresponding to the specific halogenated alkene by analyzing the reactor outlet gas with a gas chromatograph.

Silicon tetrafluoride released in the method of producing a halogenated alkene in the present disclosure can be utilized as a raw material for producing high-performance optical fibers, a gas for semiconductor production, and the like.

Further, silicon tetrafluoride released out of the reaction system can be reacted with water or alkali and recovered as hydrogen fluoride or fluoride salts. These recovered compounds can be used as etching agents or utilized as raw materials for organic fluorine compounds.

Note that, for example, to convert calcium fluoride ($CaF_2$) generated by a conventional method using calcium carbonate ($CaCO_3$) as a catalyst into hydrogen fluoride, radical conditions of reacting with sulfuric acid are required, and pre-treatment such as crushing $CaF_2$ which is a solid is required.

(Modifications)

As a modification, the method of producing a halogenated alkene in the present disclosure may convert a halogenated alkane containing a fluorine atom and having from 2 to 4 carbon atoms into a halogenated alkene containing a fluorine atom and having from 2 to 4 carbon atoms in a gas phase in the presence of boron oxide. The halogenated alkane and halogenated alkene in this case are the same as those described above. The same applies to diluent gases, reactors, and the like that can be used.

Boron oxide may be used in combination with other components, and may be in the form of, for example, borosilicate glass.

Examples

Hereinafter, the present disclosure will be described more specifically by way of examples, but the present disclosure is not limited to the following examples unless it exceeds the gist thereof. Examples 2, 3, 5, 7 to 20 are Working Examples, and Examples 1, 4, 6 are Comparative Examples.

(Outlet gas composition)

A product gas taken out from the outlet of the reactor (hereinafter, also referred to as "reactor outlet gas") at every specific time from the start of the reaction was analyzed by gas chromatography. Specifically, a column (product name "DB-1", manufactured by Agilent, length 60 m, inner diameter 0.25 mm, film thickness 1 μm) was attached to a gas chromatograph (product name "GC6850", manufactured by Agilent) and analysis was performed. Tables show the area

ratio (GC Area%) of the reactor outlet gas.

Further, the obtained area ratio (GC Area%) was converted based on gas chromatography relative sensitivity, and a molar composition was determined such that a total of the components described in the table was 100% by mol.

(Rate of change in production amount)

A ratio (%) of the production amount of the halogenated alkene at each reaction time was determined, with respect to the production amount of the halogenated alkene in 1 hour from the start of the reaction. Unless otherwise noted, a rate of change in the production amount of the halogenated alkene was determined using the values of the above molar composition.

[Example 1]

140 g of α-alumina (product name "N612", manufactured by JGC Catalysts and Chemicals Co., Ltd.) was filled into an Inconel 600 reaction tube having an inner diameter of 2.04 cm and a length of 30 cm, installed in a tubular electric furnace, and a dehydrofluorination reaction to HFO-1123 was carried out by circulating a 1/1 (mol/mol) mixed gas of nitrogen/HFC-134a at the flow rates shown in Table 1 at 700°C.

[Table 1]

| Reaction temperature (°C) | | | 700 | 700 | 700 | 700 | 700 | 700 |
|---|---|---|---|---|---|---|---|---|
| Flow rate (mL/min) | | HFC-134a | 200 | 200 | 200 | 200 | 200 | 200 |
| | | Nitrogen | 200 | 200 | 200 | 200 | 200 | 200 |
| Reaction continuation time (h) | | | 1.0 | 2.0 | 3.0 | 4.0 | 5.0 | 6.0 |
| Outlet gas composition | GCArea% | HFO-1123 | 59 | 60 | 17 | 3.2 | 2.2 | 1.8 |
| | | HFC-134a | 25 | 38 | 82 | 93 | 92 | 91 |
| | % by mol | HFO-1123 | 80 | 72 | 25 | 5.4 | 3.9 | 3.2 |
| | | HFC-134a | 20 | 28 | 75 | 95 | 96 | 97 |
| Rate of change in production amount (%) | | | 100 | 90 | 31 | 6.8 | 4.9 | 4.0 |

[Example 2]

In the same manner as in Example 1, except that α-alumina was changed to glass beads 1 (Unibeads series, manufactured by Unitika Glass Beads Co., Ltd.), a dehydrofluorination reaction was carried out.

[Table 2]

| Reaction temperature (°C) | | | 700 | 700 | 700 | 700 | 700 | 700 |
|---|---|---|---|---|---|---|---|---|
| Flow rate (mL/min) | | HFC-134a | 200 | 200 | 200 | 200 | 200 | 200 |
| | | Nitrogen | 200 | 200 | 200 | 200 | 200 | 200 |
| Reaction continuation time (h) | | | 1.0 | 2.0 | 3.0 | 4.0 | 4.5 | 5.0 |
| Outlet gas compositio n | GCArea% | HFO-1123 | 13 | 10 | 9.0 | 8.0 | 7.7 | 7.8 |
| | | HFC-134a | 84 | 86 | 88 | 90 | 91 | 91 |
| | % by mol | HFO-1123 | 20 | 16 | 15 | 13 | 12 | 12 |
| | | HFC-134a | 80 | 84 | 85 | 87 | 88 | 88 |
| Rate of change in production amount (%) | | | 100 | 80 | 75 | 65 | 60 | 60 |

Comparing Example 1 and Example 2, in Example 1 using α-alumina, the production amount of halogenated alkene significantly decreased 3 hours after the start of the reaction, and after 4 hours, the production amount became extremely small, whereas in Example 2 using glass beads 1, it can be seen that the decrease in the production amount is remarkably suppressed. Further, in Example 2, it can be seen that the concentration of HFO-1123 in the outlet gas

composition is stably maintained.

[Example 3]

In Example 2, the reaction was interrupted after 5 hours had elapsed from the start of the reaction, a weight of the glass beads was measured, and the reaction was restarted by replenishing 28 g of glass beads 1 corresponding to the decreased weight. An additional reaction time in Table 3 is a reaction time after restarting. Further, a rate of change in production amount (%) is a value with respect to the production amount of halogenated alkene at 1 hour from the start of the reaction in Example 2.

[Table 3]

| Reaction temperature (°C) | | | 700 | 700 | 700 | 700 |
|---|---|---|---|---|---|---|
| Flow rate (mL/min) | | HFC-134a | 200 | 200 | 200 | 200 |
| | | Nitrogen | 200 | 200 | 200 | 200 |
| Additional reaction time (h) | | | 0.5 | 1.0 | 1.5 | 2.0 |
| Outlet gas composition | GCArea% | HFO-1123 | 8.5 | 9.1 | 9.7 | 11 |
| | | HFC-134a | 89 | 89 | 88 | 86 |
| | % by mol | HFO-1123 | 14 | 15 | 15 | 18 |
| | | HFC-134a | 86 | 85 | 85 | 82 |
| Rate of change in production amount (%) | | | 70 | 75 | 75 | 90 |

It can be seen that the production amount is clearly improved and recovered by the replenishment of glass beads 1.

[Example 4]

In the same manner as in Example 1, except that HFC-134a was changed to HFC-134, a dehydrofluorination reaction to HFO-1123 was carried out.

[Table 4]

| Reaction temperature (°C) | | | 700 | 700 | 700 | 700 | 700 | 700 |
|---|---|---|---|---|---|---|---|---|
| Flow rate (mL/min) | | HFC-134 | 200 | 200 | 200 | 200 | 200 | 200 |
| | | Nitrogen | 200 | 200 | 200 | 200 | 200 | 200 |
| Reaction continuation time (h) | | | 1.0 | 2.0 | 2.5 | 3.0 | 3.5 | 4.0 |
| Outlet gas composition | GCArea% | HFO-1123 | 42 | 38 | 17 | 3.5 | 0.56 | 0.31 |
| | | HFC-134a | 17 | 14 | 8.1 | 3.3 | 1.9 | 1.9 |
| | | HFC-134 | 0.63 | 34 | 65 | 87 | 93 | 94 |
| | % by mol | HFO-1123 | 80 | 55 | 27 | 5.8 | 0.91 | 0.51 |
| | | HFC-134a | 19 | 13 | 7.8 | 3.3 | 1.9 | 1.8 |
| | | HFC-134 | 0.8 | 32 | 66 | 91 | 97 | 98 |
| Rate of change in production amount (%) | | | 100 | 69 | 34 | 7.3 | 1.1 | 0.64 |

[Example 5]

In the same manner as in Example 2, except that HFC-134a was changed to HFC-134, a dehydrofluorination reaction to HFO-1123 was carried out.

[Table 5]

| Reaction temperature (°C) | | | 700 | 700 | 700 | 700 | 700 |
|---|---|---|---|---|---|---|---|
| Flow rate (mL/min) | | HFC-134 | 200 | 200 | 200 | 200 | 200 |
| | | Nitrogen | 200 | 200 | 200 | 200 | 200 |
| Reaction continuation time (h) | | | 1.0 | 2.0 | 3.0 | 4.0 | 5.0 |
| Outlet gas composition | GCArea% | HFO-1123 | 24 | 23 | 23 | 19 | 15 |
| | | HFC-134a | 0.14 | 0.11 | - | - | 0.071 |
| | | HFC-134 | 75 | 75 | 75 | 79 | 83 |
| | % by mol | HFO-1123 | 33 | 33 | 33 | 28 | 22 |
| | | HFC-134a | 0 | 0 | 0 | 0 | 0 |
| | | HFC-134 | 67 | 67 | 67 | 72 | 78 |
| Rate of change in production amount (%) | | | 100 | 100 | 100 | 85 | 67 |

In the table, "-" means that the corresponding component was below the detection limit.

Comparing Example 4 and Example 5, in Example 4 using $\alpha$-alumina, a production amount of halogenated alkene significantly decreased 2.5 hours after the start of the reaction, and almost no generation was observed after 3.5 hours, whereas in Example 5 using glass beads 1, it can be seen that the decrease in a production amount is remarkably suppressed.

Further, in Example 4 using $\alpha$-alumina, HFC-134a, which is a by-product, was generated in an amount above a certain level, whereas in Example 5 using glass beads 1, almost no HFC-134a was produced.

Further, in Example 5, it can be seen that the concentration of HFO-1123 in the outlet gas composition is stably maintained.

[Example 6]

In the same manner as in Example 1, except that HFC-134a was changed to HFC-125, the diluent gas was changed to difluoromethane (R32), and a 1/1 (mol/mol) mixed gas of R32/HFC-125 was circulated at 400 mL/min, a dehydrofluorination reaction to FO-1114 was carried out. An amount of change in a production amount in Table 6 was determined from the area ratio (GC Area%) of the reactor outlet gas.

[Table 6]

| Reaction temperature (°C) | | 700 | 700 | 700 | 700 | 700 | 700 |
|---|---|---|---|---|---|---|---|
| Flow rate (mL/min) | HFC-125 +R32 | 400 | 400 | 400 | 400 | 400 | 400 |
| Reaction continuation time (h) | | 1.0 | 1.5 | 2.0 | 3.0 | 4.0 | 5.0 |
| GCArea% | FO-1114 | 4.9 | 2.1 | 0.81 | 0.36 | 0.22 | 0.16 |
| | HFC-125 +R32 | 42 | 75 | 97 | 99 | 99 | 99 |
| Rate of change in production amount (%) | | 100 | 43 | 17 | 7.3 | 4.4 | 3.3 |

[Example 7]

In the same manner as in Example 6, except that $\alpha$-alumina was changed to glass beads 1 of Example 2, a dehydrofluorination reaction was carried out. An amount of change in the production amount in Table 7 was determined from the area ratio (GC Area%) of the reactor outlet gas.

[Table 7]

| Reaction temperature (°C) | | 700 | 700 | 700 | 700 | 700 | 700 |
|---|---|---|---|---|---|---|---|
| Flow rate (mL/min) | HFC-125 +R32 | 400 | 400 | 400 | 400 | 400 | 400 |
| Reaction continuation time (h) | | 1.0 | 1.5 | 2.0 | 3.0 | 4.0 | 5.0 |

(continued)

| | | | | | | | |
|---|---|---|---|---|---|---|---|
| GCArea% | FO-1114 | 1.5 | 1.5 | 1.4 | 1.4 | 1.3 | 1.1 |
| | HFC-125 +R32 | 98 | 98 | 98 | 98 | 98 | 99 |
| Rate of change in production amount (%) | | 100 | 100 | 93 | 93 | 87 | 73 |

Comparing Example 6 and Example 7, in Example 6 using α-alumina, the production amount of halogenated alkene significantly decreased 2 hours after the start of the reaction, and after 4 hours, the production amount became extremely small, whereas in Example 7 using glass beads 1, it can be seen that the decrease in the production amount is remarkably suppressed.

In Example 6, the selectivity of the compounds listed in the table was also low in the early stage of the reaction start.

In Example 7, it can be seen that the concentration of FO-1114 in the outlet gas composition is stably maintained.

[Example 8]

In the same manner as in Example 6, except that α-alumina was changed to silica sand and sodium fluoride mixed at a mass ratio of 1/1 (70 g / 70 g), a dehydrofluorination reaction was carried out.

[Table 8]

| Reaction temperature (°C) | | | 700 | 700 | 700 | 700 | 700 |
|---|---|---|---|---|---|---|---|
| Flow rate (mL/min) | | HFC-134a | 200 | 200 | 200 | 200 | 200 |
| | | Nitrogen | 200 | 200 | 200 | 200 | 200 |
| Reaction continuation time (h) | | | 1.0 | 2.0 | 3.0 | 4.0 | 5.0 |
| Outlet gas composition | GCArea% | HFO-1123 | 4.6 | 4.1 | 4.0 | 3.9 | 3.8 |
| | | HFC-134a | 95 | 95 | 96 | 96 | 96 |
| | % by mol | HFO-1123 | 7.3 | 6.6 | 6.4 | 6.3 | 6.1 |
| | | HFC-134a | 93 | 93 | 94 | 94 | 94 |
| Rate of change in production amount (%) | | | 100 | 90 | 88 | 86 | 84 |

[Example 9]

In the same manner as in Example 8, except that sodium fluoride was changed to potassium fluoride, a dehydrofluorination reaction was carried out.

[Table 9]

| Reaction temperature (°C) | | | 700 | 700 | 700 | 700 | 700 |
|---|---|---|---|---|---|---|---|
| Flow rate (mL/min) | | HFC-134a | 200 | 200 | 200 | 200 | 200 |
| | | Nitrogen | 200 | 200 | 200 | 200 | 200 |
| Reaction continuation time (h) | | | 1.0 | 2.0 | 3.0 | 4.0 | 5.0 |
| Outlet gas composition | GCArea% | HFO-1123 | 18 | 14 | 13 | 12 | 11 |
| | | HFC-134a | 80 | 85 | 86 | 87 | 88 |
| | % by mol | HFO-1123 | 27 | 22 | 19 | 18 | 18 |
| | | HFC-134a | 73 | 78 | 81 | 82 | 82 |
| Rate of change in production amount (%) | | | 100 | 81 | 70 | 67 | 67 |

[Example 10]

In the same manner as in Example 9, except that a mass ratio of silica sand and potassium fluoride was changed

to 5/2, a dehydrofluorination reaction was carried out.

[Table 10]

| Reaction temperature (°C) | | 700 | 700 | 700 | 700 | 700 | |
|---|---|---|---|---|---|---|---|
| Flow rate (mL/min) | HFC-134a | 200 | 200 | 200 | 200 | 200 | |
| | Nitrogen | 200 | 200 | 200 | 200 | 200 | |
| Reaction continuation time (h) | | 1.0 | 2.0 | 3.0 | 4.0 | 5.0 | |
| Outlet gas composition | GCArea% | HFO-1123 | 8.6 | 7.1 | 7.4 | 6.8 | 6.2 |
| | | HFC-134a | 91 | 93 | 92 | 93 | 93 |
| | % by mol | HFO-1123 | 14 | 11 | 12 | 11 | 10 |
| | | HFC-134a | 86 | 89 | 88 | 89 | 90 |
| Rate of change in production amount (%) | | | 100 | 79 | 86 | 79 | 71 |

Comparing Example 1 and Examples 8, 9, and 10, in Example 1 using α-alumina, the production amount of halogenated alkene significantly decreased 3 hours after the start of the reaction, and after 4 hours, the production amount became extremely small, whereas in Examples 8, 9, and 10 using a silicon oxide compound, it can be seen that the decrease in the production amount is remarkably suppressed.

[Example 11]

In the same manner as in Example 2, except that the reaction temperature was changed as shown in Table 11, a dehydrofluorination reaction from HFC-134a to HFO-1123 was carried out. Table 11 shows the outlet gas composition 0.5 hours after the start of the reaction.

[Table 11]

| Reaction temperature (°C) | | | 600 | 700 | 800 | 900 |
|---|---|---|---|---|---|---|
| Flow rate (mL/min) | | HFC-134a | 200 | 200 | 200 | 200 |
| | | Nitrogen | 200 | 200 | 200 | 200 |
| Outlet gas composition | GCArea% | HFO-1123 | 2.9 | 12 | 13 | 19 |
| | | HFC-134a | 96 | 86 | 84 | 73 |
| | % by mol | HFO-1123 | 4.7 | 19 | 21 | 31 |
| | | HFC-134a | 95 | 81 | 79 | 69 |

[Example 12]

In the same manner as in Example 5, except that the reaction temperature was changed as shown in Table 12, a dehydrofluorination reaction from HFC-134 to HFO-1123 was carried out. Table 12 shows the outlet gas composition 0.5 hours after the start of the reaction.

[Table 12]

| Reaction temperature (°C) | | 600 | 650 | 700 | 750 | 800 | 850 |
|---|---|---|---|---|---|---|---|
| Flow rate (mL/min) | HFC-134 | 200 | 200 | 200 | 200 | 200 | 200 |
| | Nitrogen | 200 | 200 | 200 | 200 | 200 | 200 |

(continued)

| Reaction temperature (°C) | | | 600 | 650 | 700 | 750 | 800 | 850 |
|---|---|---|---|---|---|---|---|---|
| Outlet gas composition | GCArea% | HFO-1123 | 1.3 | 7.7 | 17 | 36 | 43 | 3.4 |
| | | HFC-134a | 0.07 | 0.10 | 0.19 | 0.46 | 0.63 | 0 |
| | | HFC-134 | 98 | 91 | 80 | 58 | 51 | 4.9 |
| | % by mol | HFO-1123 | 2.0 | 12 | 25 | 50 | 56 | 52 |
| | | HFC-134a | 0 | 0 | 0 | 0 | 0.51 | 0 |
| | | HFC-134 | 98 | 88 | 75 | 50 | 43 | 48 |

[Example 13]

In the same manner as in Example 8, except that sodium fluoride was changed to sodium chloride, a dehydrofluorination reaction was carried out. Table 13 shows the outlet gas composition 1.0 hour after the start of the reaction.

[Example 14]

In the same manner as in Example 8, except that sodium fluoride (70 g) was changed to lithium fluoride (35 g) and a mixture of silica sand (70 g) and lithium fluoride (35 g) was used, a dehydrofluorination reaction was carried out. Table 13 shows the outlet gas composition 1.0 hour after the start of the reaction.

[Table 13]

| Example | | | Example 13 | Example 14 |
|---|---|---|---|---|
| Reaction temperature (°C) | | | 700 | 700 |
| Flow rate (mL/min) | | HFC-134a | 200 | 200 |
| | | Nitrogen | 200 | 200 |
| Outlet gas composition | GCArea% | HFO-1123 | 1.3 | 1.4 |
| | | HFC-134a | 87 | 98 |
| | % by mol | HFO-1123 | 2.4 | 2.3 |
| | | HFC-134a | 98 | 98 |

[Example 15]

In the same manner as in Example 8, except that sodium fluoride was changed to cesium fluoride, a dehydrofluorination reaction was carried out. Table 14 shows the outlet gas composition 0.5 hours after the start of the reaction.

[Table 14]

| Reaction temperature (°C) | | | 500 | 550 | 600 | 650 |
|---|---|---|---|---|---|---|
| Flow rate (mL/min) | | HFC-134a | 200 | 200 | 200 | 200 |
| | | Nitrogen | 200 | 200 | 200 | 200 |
| Outlet gas composition | GCArea% | HFO-1123 | 3.3 | 15 | 42 | 44 |
| | | HFC-134a | 97 | 85 | 57 | 52 |
| | % by mol | HFO-1123 | 5.4 | 22 | 54 | 58 |
| | | HFC-134a | 95 | 78 | 45 | 41 |

[Example 16]

In the same manner as in Example 2, except that the total flow rate and ratio of the circulating gas were changed as shown in Table 15, a dehydrofluorination reaction from HFC-134a to HFO-1123 was carried out. Table 15 shows the outlet gas composition 0.25 hours after the start of the reaction.

[Table 15]

| Reaction temperature (°C) | | | 700 | 700 | 700 | 700 | 700 | 700 | 700 |
|---|---|---|---|---|---|---|---|---|---|
| Flow rate (mL/min) | | HFC-134a | 100 | 50 | 30 | 11 | 50 | 300 | 333 |
| | | Nitrogen | 100 | 50 | 30 | 11 | 0 | 300 | 333 |
| Outlet gas composition | GCArea% | HFO-1123 | 15 | 19 | 26 | 4.8 | 21 | 14 | 14 |
| | | HFC-134a | 80 | 72 | 55 | 13 | 66 | 83 | 83 |
| | % by mol | HFO-1123 | 23 | 30 | 44 | 39 | 34 | 22 | 22 |
| | | HFC-134a | 77 | 70 | 56 | 61 | 66 | 78 | 78 |

[Example 17]

In the same manner as in Example 2, except that HFC-134a was changed to HFC-152a, a dehydrofluorination reaction to HFO-1141 was carried out. Table 16 shows the outlet gas composition 0.5 hours after the start of the reaction.

[Example 18]

In the same manner as in Example 17, except that the glass beads were changed to a mixture of silica sand (120 g) and potassium fluoride (20 g), a dehydrofluorination reaction to HFO-1141 was carried out. Table 16 shows the outlet gas composition 0.5 hours after the start of the reaction.

[Table 16]

| Example | | Example 17 | | | | | Example 18 |
|---|---|---|---|---|---|---|---|
| Reaction temperature (°C) | | 500 | 550 | 600 | 650 | 700 | 600 |
| Flow rate (mL/min) | HFC-152a | 200 | 200 | 200 | 200 | 200 | 200 |
| | Nitrogen | 200 | 200 | 200 | 200 | 200 | 200 |
| GCArea% | HFO-1141 | 2.4 | 8.1 | 24 | 46 | 57 | 13 |
| | HFC-152a | 96 | 89 | 70 | 43 | 14 | 87 |

[Example 19]

In the same manner as in Example 2, except that HFC-134a was changed to HFC-143, a dehydrofluorination reaction to HFO-1132 and 1132a was carried out. Table 17 shows the outlet gas composition 0.5 hours after the start of the reaction.

[Example 20]

In the same manner as in Example 19, except that the glass beads were changed to a mixture of silica sand (120 g) and potassium fluoride (20 g), a dehydrofluorination reaction was carried out. Table 17 shows the outlet gas composition 0.5 hours after the start of the reaction.

[Table 17]

| Example | Example 19 | Example 20 |
|---|---|---|
| Reaction temperature (°C) | 600 | 600 |

(continued)

| Example | | Example 19 | Example 20 |
|---|---|---|---|
| Flow rate (mL/min) | HFC-143 | 200 | 200 |
| | Nitrogen | 200 | 200 |
| GCArea% | HFO-1132a | 9.2 | 9.5 |
| | HFO-1132 | 5.5 | 8.1 |
| | HFC-143 | 28 | 67 |

The disclosure of Japanese Patent Application No. 2023-190304 is incorporated herein by reference in its entirety.

All documents, patent applications, and technical standards described in this specification are incorporated herein by reference to the same extent as if each individual document, patent application, and technical standard was specifically and individually indicated to be incorporated by reference.

## Claims

1. A method of producing a halogenated alkene, the method comprising converting a halogenated alkane containing a fluorine atom and having from 2 to 4 carbon atoms into a halogenated alkene containing a fluorine atom and having from 2 to 4 carbon atoms in a gas phase in the presence of silicon oxide and an alkali metal element.

2. The method of producing a halogenated alkene according to claim 1, wherein the halogenated alkane comprises a halogenated alkane represented by the following Formula (1), and the halogenated alkene comprises a halogenated alkene represented by the following Formula (2):

$$CR^1R^2X^1\text{-}CR^3R^4X^2 \qquad (1)$$

$$CR^1R^2{=}CR^3R^4 \qquad (2)$$

in which, in Formula (1) and Formula (2), $R^1$ to $R^4$ are each independently a hydrogen atom, a fluorine atom, a methyl group, a fluorinated methyl group, an ethyl group, or a fluorinated ethyl group, a total number of fluorine atoms in $R^1$ to $R^4$ is 1 or more, and the number of carbon atoms is from 2 to 4, and in which in Formula (1), one of $X^1$ or $X^2$ is a hydrogen atom, and another is a fluorine atom.

3. The method of producing a halogenated alkene according to claim 1 or 2, wherein silicon tetrafluoride is produced.

4. The method of producing a halogenated alkene according to claim 1 or 2, the method comprising:

producing the halogenated alkene and hydrogen fluoride by a dehydrofluorination reaction of the halogenated alkane in a gas phase; and
producing silicon tetrafluoride by a reaction between the produced hydrogen fluoride and silicon oxide.

5. The method of producing a halogenated alkene according to claim 1 or 2, wherein the halogenated alkane is at least one selected from the group consisting of 1,1-difluoroethane, 1,2-difluoroethane, 1,1,1-trifluoroethane, 1,1,2-trifluoroethane, 1,1,2,2-tetrafluoroethane, 1,1,1,2-tetrafluoroethane, and 1,1,1,2,2-pentafluoroethane.

6. The method of producing a halogenated alkene according to claim 1 or 2, wherein the halogenated alkene is at least one selected from the group consisting of fluoroethylene, 1,1-difluoroethylene, 1,2-difluoroethylene, trifluoroethylene, and tetrafluoroethylene.

7. The method of producing a halogenated alkene according to claim 1 or 2, wherein the halogenated alkane is converted in the presence of a diluent gas.

8. The method of producing a halogenated alkene according to claim 7, wherein the diluent gas is at least one selected from the group consisting of nitrogen, hydrogen, carbon dioxide, helium, ethane, propane, isobutane, n-butane,

propylene, and fluorinated methane.

9. The method of producing a halogenated alkene according to claim 1 or 2, wherein the halogenated alkane is converted at a temperature of from 400 to 1000°C.

## INTERNATIONAL SEARCH REPORT

International application No. **PCT/JP2024/031785**

**A. CLASSIFICATION OF SUBJECT MATTER**

***C07C 17/25***(2006.01)i; ***C07B 61/00***(2006.01)i; ***C07C 21/18***(2006.01)i; ***C07C 21/185***(2006.01)i
FI: C07C17/25; C07C21/18; C07C21/185; C07B61/00 300

According to International Patent Classification (IPC) or to both national classification and IPC

**B. FIELDS SEARCHED**

Minimum documentation searched (classification system followed by classification symbols)

C07B31/00-63/04, C07C1/00-409/44

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | WO 2023/171275 A1 (KUREHA CORP.) 14 September 2023 (2023-09-14) claims, paragraph [0014], example 1 | 1-9 |
| X | US 2697124 A (THE M. W. KELLOGG COMPANY) 14 December 1954 (1954-12-14) table 1 (example no. 9, 10) | 1, 3, 4, 7-9 |
| X | JP 03-504684 A (THE DOW CHEMICAL COMPANY) 17 October 1991 (1991-10-17) claims, p. 3, upper right column, line 19 to lower left column, line 21, p. 4, upper left column, line 24 to upper right column, line 10, example 12 | 1-9 |
| A | JP 03-264543 A (DAIKIN INDUSTRIES, LTD.) 25 November 1991 (1991-11-25) | 1-9 |
| A | JP 62-26239 A (E. I. DU PONT DE NEMOURS AND CO.) 04 February 1987 (1987-02-04) | 1-9 |
| A | US 2022/0219146 A1 (XI'AN MODERN CHEMISTRY RESEARCH INSTITUTE) 14 July 2022 (2022-07-14) | 1-9 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 October 2024** | **19 November 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/031785**

| Patent document cited in search report | Publication date (day/month/year) | Patent family member(s) | Publication date (day/month/year) |
|---|---|---|---|
| WO 2023/171275 A1 | 14 September 2023 | (Family: none) | |
| US 2697124 A | 14 December 1954 | (Family: none) | |
| JP 03-504684 A | 17 October 1991 | US 4816609 A<br>claims, column 3, lines 23-49<br>WO 1990/011131 A1<br>EP 389650 A1<br>KR 10-1992-0700067 A | |
| JP 03-264543 A | 25 November 1991 | (Family: none) | |
| JP 62-26239 A | 04 February 1987 | US 4814522 A<br>EP 203807 A1 | |
| US 2022/0219146 A1 | 14 July 2022 | WO 2020/224663 A1<br>CN 110013853 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017104829 A
- JP 2023190304 A